# EUROPÄISCHE PATENTSCHRIFT

(11) **EP 0 944 838 B1**
(45) Veröffentlichungstag und Bekanntmachung des Hinweises auf die Patenterteilung: **10.09.2003**
(21) Anmeldenummer: 97951977.4
(22) Anmeldetag: 26.11.1997
(51) Int. Cl.: G01N 33/68, G01N 33/543

(54) **ANTIGENSPEZIFISCHER IgG-NACHWEIS**
ANTIGEN-SPECIFIC IgG DETECTION
DETECTION D'IgG SPECIFIQUES D'ANTIGENES

(30) Priorität: 29.11.1996 DE 19649390
(43) Veröffentlichungstag der Anmeldung: 29.09.1999
(73) Patentinhaber: Roche Diagnostics GmbH, 68298 Mannheim (DE)
(72) Erfinder: FAATZ, Elke, D-82386 Huglfing (DE); SCHMITT, Urban, D-82386 Oberhausen (DE)
(86) Internationale Anmeldenummer: EP9706582
(87) Internationale Veröffentlichungsnummer: WO98023961

(56) Entgegenhaltungen:
- EP-A- 0 366 092
- EP-A- 0 507 587
- DE-A- 3 820 556
- US-A- 4 929 543
- US-A- 5 362 655

## Beschreibung

Die Erfindung betrifft ein Verfahren zur Bestimmung von antigenspezifischen Antikörpern der Immunglobulinklasse G in Körperflüssigkeiten durch Inkubation der Probe mit mindestens zwei verschiedenen Rezeptoren R₁ und R₂, wobei beide Rezeptoren mit dem Antikörper spezifisch bindefähig sind, R₁ an eine feste Phase gebunden ist oder gebunden werden kann und R₂ eine Markierung trägt, Trennung der festen von der flüssigen Phase und Messung der Markierung, wobei als R₂ ein Konjugat aus einem vom zu bestimmenden Antikörper spezifisch erkannten Bindepartner in monomerer Form und einer Markierung verwendet wird.

Insbesondere betrifft die Erfindung eine Methode zum spezifischen Nachweis von Immunglobulinen der Klasse IgG in Anwesenheit von Immunglobulinen der Klasse IgM.

Das Immunsystem eines Säugetierorganismus produziert als Antwort auf das Einbringen fremder Substanzen Antikörper, die auch Immunglobuline genannt werden. Sie dienen zur Abwehr der Fremdsubstanzen, die auch als Antigene bezeichnet werden. Die Immunglobuline können in fünf verschiedene Klassen eingeteilt werden. Man unterscheidet Immunglobuline der Klassen M, G, A, E und D. Diese fünf Immunglobulinklassen unterscheiden sich jeweils in der Zusammensetzung der schweren Kette, die als µ-, γ-, α-, ε- bzw. δ-Kette bezeichnet wird.

Jede Immunglobulinklasse hat im Organismus eine andere Aufgabe. Die Immunglobuline der Klasse M treten bei einem ersten Kontakt mit dem Antigen, der sogenannten Erstimmunisierung, auf. Die Konzentration dieser Immunglobuline nimmt jedoch bei fortschreitender Infektion schnell wieder ab. Die Immunglobuline der Klasse G werden bei einer ersten Immunisierung erst langsam gebildet und treten bei einer zweiten Infektion mit demselben Antigen in großen Mengen auf. Die Immunglobuline der Klasse A sind auf den Schleimhautoberflächen des Organismus anzutreffen und sind für die dortigen Abwehrvorgänge zuständig.

Die Immunglobuline der Klasse E sind hauptsächlich für allergische Reaktionen verantwortlich. Die genaue Funktion der Immunglobuline der Klasse D ist bislang nicht bekannt.

Die einzelnen Immunglobulinklassen kommen im Blut in sehr unterschiedlichen Konzentrationen vor. So sind die Immunglobuline der Klasse G (IgG) in normalem menschlichen Serum mit einem Anteil von etwa 75 %, das entspricht einem Serumgehalt von 8 bis 18 mg/ml, die am stärksten vertretene Klasse. Das am zweithäufigsten auftretende Immunglobulin ist das IgA, dessen durchschnittliche Serumkonzentration 0,9 bis 4,5 mg/ml beträgt. Immunglobuline der Klasse M sind in einer Konzentration von 0,6 bis 2,8 mg/ml, Immunglobuline der Klasse D von 0,003 bis 0,4 mg/ml vorhanden. Am geringsten ist der Anteil von IgE-Antikörpern, die nur in einer Konzentration von 0,02 bis 0,05 µg/ml im Serum auftreten.

Für die differenzierte Diagnostik vieler Erkrankungen ist es wichtig, den Nachweis für Antikörper einer oder mehrerer ganz bestimmter Immunglobulinklassen, die für ein bestimmtes Antigen spezifisch sind, zu führen. Nur mittels eines klassenspezifischen Antikörper-Nachweises, bzw. durch das Ausschließen der Anwesenheit bestimmter Immunglobulinklassen (z.B. Detektion von IgG- und IgA-Antikörpern, aber keine Detektion von IgM-Antikörpem) kann eine befriedigende Diagnose bei viralen, bakteriellen und parasitären Infektionen sichergestellt werden. Dies ist besonders wichtig zur Unterscheidung frischer bzw. akuter und weiter zurückliegender Infektionen sowie zur klinischen Kontrolle des Verlaufs einer Infektion. Der klassenspezifische Nachweis von Antikörpern ist insbesondere wichtig bei HIV-, Hepatitis A-, Hepatitis B-, Toxoplasmose-, Rubella- und Chlamydia-Infektionen. Ebenso ist der klassenspezifische Nachweis von für ein bestimmtes Antigen spezifischen Antikörpern bei der Bestimmung des Titers schützender Antikörper und zur Überprüfung des Impferfolges notwendig. Es besteht daher aus diagnostischer Sicht ein großes Interesse, insbesondere Antikörper der nicht akuten Infektionsstadien wie IgG- und IgA-Antikörper nachzuweisen.

Im Stand der Technik werden verschiedene Methoden zum Nachweis von für ein Antigen spezifischen Antikörpern einer bestimmten Klasse beschrieben. So wird der Nachweis von antigenspezifischen Antikörpern einer bestimmten Klasse in einer Probe häufig dadurch geführt, daß die spezifischen Antikörper an eine mit dem spezifischen Antigen beschichtete Festphase gebunden werden. Der Nachweis der für das Antigen spezifischen, nun an die Festphase gebundenen Immunglobuline (Ig) erfolgt durch die Bindung von Antikörpern, die spezifisch gegen humanes Ig einer bestimmten Klasse gerichtet sind, an die nachzuweisenden Ig-Moleküle. Die gegen humanes Ig gerichteten Antikörper sind mit einer Markierung versehen, über die die Detektion stattfindet. Eine solche Testführung ist aber nur möglich, wenn vor der Reaktion mit den klassenspezifischen, gegen humanes Ig gerichteten markierten Antikörpern durch Waschung alles unspezifische, nicht gebundene Ig entfernt wird. Eine Einschritt-Testführung, wie sie für automatisierte Systeme oft benötigt wird, ist somit nicht möglich.

Nach der im US Patent 4,292,403 beschriebenen Methode werden antigenspezifsche Antikörper einer bestimmten Immunglobulinklasse dadurch nachgewiesen, daß ein klassenspezifischer Antikörper an einer Festphase immobilisiert wird, der zu bestimmende Probenantikörper bindet, anschließend das spezifische Antigen zugesetzt wird und das gebundene Antigen durch einen weiteren für das Antigen spezifischen markierten Antikörper gebunden wird. Dieses Verfahren hat jedoch den Nachteil, daß alle Antikörper der zu bestimmenden Klasse an den klassenspezifischen immobilisierten Antikörper binden. Die Bindung der Probenantikörper erfolgt nicht antigenspezifisch. Dies kann zu Lasten der Sensitivität des Tests gehen, da möglicherweise nicht genügend freie Bindungsstellen für den antigenspezifischen Antikörper vorhanden sind. Auch bei dieser Testführung sind mehrere Wasch-Schritte nötig. Mit mit diesem Verfahren ist keine Einschritt-Testführung möglich.

Eine Möglichkeit, einen Antikörper-Nachweis in einem Einschritt-Test durchzuführen, eröffnet der sogenannte Brückentest. Das Brückentest-Konzept ist in der EP-A-0 280 211 beschrieben. Dabei wird ein erster Rezeptor, der mit dem zu bestimmenden Antikörper spezifisch bindefähig ist wie beispielsweise ein Antigen, an eine Festphase gebunden. Der zu bestimmende Antikörper bindet an das festphasengebundene Antigen. Im Testansatz ist außerdem ein weiteres, spezifisches Antigen vorhanden, das mit einer Markierung versehen ist. Der Nachweis des Antikörpers erfolgt über die Markierung. Bei diesem Test werden alle antigenspezifischen Antikörper nachgewiesen und nicht nur die Antikörper einer bestimmten Klasse.

In der EP-A-0 307 149 und im US-Patent 5,254,458 werden Verfahren nach dem Brückentest-Prinzip zum Nachweis von Antikörpern, die spezifisch gegen ein Antigen gerichtet sind, offenbart. Hierbei werden rekombinant hergestellte Peptide, die von einem bestimmten Epitop des Antigens abgeleitet sind, für die Bindung des nachzuweisenden Antikörpers eingesetzt. Ein Peptid wird an einer Festphase immobilisiert. Der Probenantikörper bindet an das Peptid. Zum Nachweis wird ein weiteres, markiertes Peptid an den Probenantikörper gebunden. Die rekombinanten Peptide werden in unterschiedlichen Organismen exprimiert, um die Spezifität des Nachweises zu erhöhen. Auch bei dieser Methode binden Antikörper aller Klassen an die Peptide. Eine Differenzierung zwischen beispielsweise IgG- und IgM-Antikörpern ist nicht möglich.

EP-A-0 386 713 beschreibt ein Verfahren zum Nachweis von Antikörpern gegen HIV unter Verwendung von zwei festen Trägern, wobei an beide festen Träger verschiedene HIV-Antigene immobilisiert werden, die jeweils mit einem Aliquot einer Probe und einem markierten HIV-Antigen in Kontakt gebracht werden, wobei das Vorhandensein von Antikörpern durch eine positive Reaktion in mindestens einem der Tests nachgewiesen wird. Als HIV-Antigene werden rekombinant hergestellte Polypeptide offenbart. In der EP-A-0 627 625 ist eine Methode auf Westernblot-Basis offenbart, bei der mittels rekombinanter Proteine oder synthetischer Peptide ebenfalls HIV-Antikörper nachgewiesen werden können. Beide Methoden ermöglichen jedoch nicht den klassenspezifischen Nachweis der antigenspezifischen Antikörper.

In der EP 0 366 092 A2 wird ein Verfahren zur Bestimmung von für ein Antigen A klassenspezifischer Antikörper offenbart, wobei die Probelösung gleichzeitig mit einem Rezeptor R¹, der mit dem zu bestimmenden Antikörper bindefähig ist, und einem Überschuss an humanen Antikörpern oder deren Fragmente, die der gleichen Immunglobulinklasse angehören, wie die zu bestimmenden Antikörper und nicht mit dem Antigen A bindefähig sind, inkubiert wird und anschließend ein Rezeptor R₂ zugegeben wird, der markiert ist und entweder mit dem zu bestimmenden Antikörper oder dem Antigen A bindefähig ist.

DE 38 20 556 A1 offenbart ein Verfahren zur Bestimmung von allergenspezifischen Antikörpern in Körperflüssigkeiten durch Inkubation mit mindestens zwei Rezeptoren R₁ und R₂, die mit den zu bestimmenden Antikörper bindefähig sind, wobei R₂ eine Markierung trägt. Als R₁ wird das mit dem zu bestimmenden Antikörper spezifisch bindefähige Allergen und als R₂ ein Konjugat aus einem gegen den Fc-Teil von IgE oder IgG gerichteten Antikörper und einer Markierung eingesetzt.

EP 0 507 587 A2 offenbart eine Methode zur Detektion eines spezifischen Immunglobulins, insbesondere IgM, in einer Probe. Hierbei wird die Probe mit einem Rezeptor für das Immunglobulin inkubiert, der an eine Festphase gebunden werden kann. Bei diesem Rezeptor handelt es sich um ein Antikörper, der gegen den Fc-Teil des Immunglobulins gerichtet ist. Weiterhin wird die Probe mit einem Antigen an das das Immunglobulin aus der Probe spezifisch bindet, und einem für das Antigen spezifischen, markierten Antikörper inkubiert. Der Nachweis erfolgt über die Markierung des antigenspezifischen Antikörpers.

Mit den bisher bekannten Verfahren ist es nicht möglich, in einem Einschrittverfahren einen antigenspezifischen Antikörper einer bestimmten Immunglobulinklasse nachzuweisen. Die aus dem Stand der Technik bekannten immunologischen Nachweisverfahren nach dem Brückentestkonzept, bei denen ein markiertes Antigen und ein an eine Festphase bindefähiges Antigen verwendet wird, ermöglichen zwar einen einstufigen Test. Bisher konnten jedoch mit diesem einfachen Prinzip Antikörper der Klassen IgG und IgM nur gemeinsam nachgewiesen werden können.

Aufgabe war es daher, eine verbesserte Methode zum Nachweis von gegen ein spezifisches Antigen gerichteten Antikörpern der nicht akuten Infektion, d.h. insbesondere der IgG-Klasse zur Verfügung zu stellen. In dem Verfahren sollte gleichzeitig sichergestellt werden, daß IgM-Antikörper gleicher Spezifität nicht nachgewiesen werden. Diese Methode sollte bevorzugt auf einem einstufigen Testprinzip bestehen, um vorteilhaft in automatisierten Systemen angewendet werden zu können.

Diese Aufgabe wird gelöst mit dem erfindungsgemäßen Verfahren zur Bestimmung eines antigenspezifischen Antikörpers der Immunglobulinklasse G durch Inkubation der Probe mit mindestens zwei verschiedenen Rezeptoren R₁ und R₂, wobei beide Rezeptoren mit dem Antikörper spezifisch bindefähig sind, R₁ an eine feste Phase gebunden ist oder gebunden werden kann und R₂ eine Markierung trägt, das dadurch gekennzeichnet ist, daß man als R₂ ein Konjugat aus einem vom zu bestimmenden Antikörper spezifisch erkannten Bindepartner in monomerer Form und einer Markierung verwendet.

Das erfindungsgemäße Verfahren erlaubt die Bestimmung von antigenspezifischen Antikörpern der Immunglobulinklasse G in Proben, in denen Antikörper der Klasse IgM gleicher Antigenspezifität vorhanden sind.

Die in der Probe vorhandenen IgA-, IgD- und IgE-Antikörper, die die gleiche Spezifität wie die nachzuweisenden IgG-Antikörper besitzen, kommen im Vergleich zu den IgG-Antikörpern in wesentlich niedrigeren Konzentrationen vor. Insbesondere die Klassen IgD und IgE sind in Konzentrationen vorhanden, die um einige Größenordnungen unter der IgG-Konzentration liegt, so daß deren Reaktivität im Nachweisverfahren das Meßergebnis nicht oder kaum verändert. IgA-Antikörper sind in Konzentrationen, die in etwa 10 % des Gesamt-IgG-Gehalts entsprechen, vorhanden. Vermutlich werden daher in diesem Verfahren auch IgA-Antikörper bestimmt. Da der Hauptzweck des Verfahrens darin besteht, Antikörper der nicht akuten Infektion nachzuweisen, ist der gemeinsame Nachweis von IgG und IgA-Antikörpern, die beide Antikörper der nicht akuten Infektion darstellen, unkritisch. Da IgG-Antikörper die am stärksten vertretene Immunglobulinklasse der nicht-akuten Infektion darstellen, wird im folgenden die Bezeichnung IgG-Nachweis verwendet. Wesentlich ist, daß IgM-Antikörper gleicher Antigenspezifität, die bei nur einer akuten Infektion verstärkt auftreten, nicht nachgewiesen werden dürfen.

Es hat sich überraschenderweise gezeigt, daß durch den erfindungsgemäßen Einsatz von Bindepartnern in monomerer Form in einem Brückentest nach dem Doppelantigen-Testprinzip ausschließlich Antikörper der Klasse IgG, die spezifisch gegen ein bestimmtes Antigen gerichtet sind, nachgewiesen werden. Antikörper der Klasse IgM mit gleicher Spezifität, die in der gleichen Probe vorhanden sind, reagieren überraschenderweise nicht oder lediglich vernachlässigbar schwach mit monomeren Peptiden und stören somit nicht den IgG-Nachweis. Der Begriff "vernachlässigbar schwach" bedeutet, daß die Antigenbindungsstellen der IgM-Antikörper nicht nachweisbar durch die Bindepartner in monomerer Form gebunden werden. Dies liegt vermutlich an der wesentlich geringeren Affinität der pentameren IgM-Antikörper zu monomer vorliegenden Epitopen im Vergleich zu den in Form einzelner Moleküle vorliegenden IgG-Antikörpern.

Bei den erfindungsgemäßen Verfahren ist deshalb eine sukzessive Testführung zur Abtrennung der IgM-Antikörper nicht unbedingt notwendig, da diese nicht stören. Ein besonderer Vorteil des Verfahrens ist daher die Einfachheit des Testablaufs.

Neben den sogenannten Naßtesten, bei denen die Testreagenzien in flüssiger Phase vorliegen, können auch alle gängigen Trockentestformate, die zum Nachweis von Proteinen bzw. Antikörpern geeignet sind, verwendet werden. Bei diesen Trockentests oder Teststreifen, wie sie beispielsweise in der EP-A-0 186 799 beschrieben sind, sind die Testkomponenten auf einem Träger aufgebracht. Wird das erfindungsgemäße Verfahren im Teststreifenformat durchgeführt, ist daher kein Wasch-Schritt notwendig. Bevorzugt wird das erfindungsgemäße Verfahren jedoch als Naßtest durchgeführt.

Es ist möglich, alle Rezeptoren und die Probe zusammen zu inkubieren und das Verfahren einstufig durchzuführen. Dies erfordert gegebenenfalls nur einen einzigen Wasch-Schritt nach der Inkubation.

Zur Durchführung des erfindungsgemäßen Verfahrens werden im Normalfall zwei verschiedene Rezeptoren R₁ und R₂ verwendet. Wird ein Naßtest verwendet, so liegt der Rezeptor R₂ in flüssiger Phase vor. R₁ kann in flüssiger Phase oder bereits an die Festphase gebunden vorliegen. Sind R₁ und R₂ in flüssiger Phase vorhanden, so liegen sie bevorzugt in gleicher Konzentration vor. Als gut geeignet haben sich Konzentrationsverhältnisse von R₁ : R₂ von 0,5 : 1,0 bis 1,0 : 5,0 erwiesen. Die optimalen Konzentrationsverhältnisse können vom Fachmann leicht ausgetestet werden.

Wird als R₁ ein mit einer Festphase bindefähiger Rezeptor eingesetzt, der noch nicht an der Festphase gebunden ist, so wird die Probe mit den Rezeptoren R₁ und R₂ inkubiert. Der Probenantikörper bindet dabei an R₁ und R₂. Diese Inkubation kann in Anwesenheit der Festphase stattfinden. Es bildet sich dabei ein Komplex aus Festphase-R₁-Probenantikörper-R₂. Anschließend wird die feste von der flüssigen Phase getrennt, gegebenenfalls die feste Phase gewaschen und die Markierung von R₂ gemessen. Die Markierung wird zumeist in der festen Phase gemessen, sie kann jedoch auch in der flüssigen Phase bestimmt werden.

Wird die Inkubation der Probe mit R₁ und R₂ in Abwesenheit der Festphase durchgeführt, so muß der gesamte Testansatz anschließend mit der Festphase in Kontakt gebracht, gegebenenfalls die Waschung durchgeführt und die Markierung gemessen werden.

Liegt der Rezeptor R₁ bereits in festphasengebundener Form vor, so werden die Probe und der Rezeptor R₂ zum festphasengebundenen Rezeptor R₁ dazugegeben und zusammen inkubiert. Das weitere Vorgehen entspricht dem oben angegebenen Verfahren.

Es ist allerdings auch möglich, das erfindungsgemäße Verfahren in mehreren Stufen durchzuführen. In diesem Fall wird zweckmäßig die Probe zunächst mit den Rezeptoren R₁ und R₂ inkubiert. Der Komplex aus R₁, R₂ und zu bestimmendem Antikörper entstandene Komplex wird anschließendend mit weiteren Rezeptoren inkubiert, wobei dies in mehreren Stufen erfolgen kann. Der weitere Testablauf entspricht dem vorher beschriebenen Verfahren.

Der Rezeptor R₂ ist ein Konjugat aus einem Bindepartner in monomerer Form und einer Markierung. Die erfindungsgemäßen Bindepartner in monomerer Form enthalten genau einen Epitopbereich bzw. nur eine Bindungsstelle für den zu bestimmenden Antikörper, d.h. eine immunologisch spezifisch mit dem zu bestimmenden IgG-Antikörper reagierende Struktur. Die monomere Struktur des Bindepartners ist wichtig, um zu gewährleisten, daß nur die nachzuweisenden antigenspezifischen IgG-Antikörper an die Bindepartner in monomerer Form binden und nicht die störenden IgM-Antikörper gleicher Spezifität.

Der Epitopbereich kann beispielsweise aus einem Antigen oder einem Antiidiotyp-Antikörper abgeleitet sein. Die Epitopbereiche können bei den Bindepartnern in monomerer Form auch von Zucker- und/oder Lipid-Strukturen oder kombinierten Strukturen mit peptidischem, lipidischen und/oder Zuckeranteil abgeleitet sein. Es können alle von einem Epitopbereich abgeleiteten Strukturen verwendet werden, die eine Bindungsstelle aufweisen, an die der nachzuweisende Antikörper der Klasse IgG in Anwesenheit von IgM-Antikörpern gleicher Spezifität spezifisch bindet. Die einzige Bedingung für die Bindungstelle, d.h. für den eingesetzten Bindepartner in monomerer Form ist, daß die spezifische Bindefähigkeit zu IgG erhalten bleibt. Diese Bedingung gilt auch für den Fall, daß Zucker- oder Lipidstrukturen in der Bindungstelle enthalten sind.

Erfindungsgemäß können auch Bindepartner in monomerer Form eingesetzt werden, die die Bindungsstelle, an die der nachzuweisende IgG-Antikörper spezifisch bindet, flankieren oder überlappen. Somit ist es möglich, auch kreuzreagierende IgG-Antikörper nachzuweisen, deren Bindungsstelle mit dem nachzuweisenden Epitop überlappt. Bevorzugt wird zum Nachweis der antigenspezifischen IgG-Antikörper daher ein Gemisch aus Bindepartnern in monomerer Form eingesetzt.

Bevorzugt werden Peptide als Bindepartner in monomerer Form eingesetzt. Unter einer Bindungsstelle ist im Falle eines Proteins als Analyt ein Peptid zu verstehen, dessen Sequenz ein Teil der Proteinsequenz eines Proteinantigens ist und an das ein gegen dieses Protein gerichteter Antikörper, der in der vorliegenden Erfindung ein IgG-Antikörper ist, spezifisch bindet. Neben diesen Peptiden sind unter einer Bindungsstelle auch noch Peptide mit Aminosäuresequenzen zu verstehen, die eine im Wesentlichen äquivalente Spezifität und/oder Affinität der Bindung an den nachzuweisenden IgG-Antikörper wie die zuvor genannten Peptide zeigen. Diese Peptide können vorzugsweise durch Substitution, Deletion oder Insertion einzelner Aminosäurereste aus den zuvor genannten Peptiden abgeleitet werden.

Es können alle Peptide verwendet werden, die eine Bindungsstelle aufweisen, an die der zu bestimmende Antikörper der Klasse IgG auch in Anwesenheit von IgM-Antikörpern gleicher Spezifität spezifisch bindet. Die einzige Bedingung für die Bindungstelle, d.h. für das eingesetzte Peptid ist, daß die spezifische Bindefähigkeit zu IgG erhalten bleibt. Unter einer Bindungsstelle ist ein Peptid zu verstehen, dessen Sequenz ein Teil der Proteinsequenz eines Proteinantigens (Analyts) ist und an das ein gegen dieses Protein gerichteter Antikörper, der in der vorliegenden Erfindung ein IgG-Antikörper ist, spezifisch bindet. Neben diesen Peptiden sind unter einer Bindungsstelle auch noch Peptide mit Aminosäuresequenzen zu verstehen, die eine im wesentlichen äquivalente Spezifität und/oder Affinität der Bindung an den zu bestimmenden IgG-Antikörper wie die zuvor genannten Peptide zeigen. Diese Peptide können vorzugsweise durch Substitution, Deletion oder Insertion einzelner Aminosäurereste aus den zuvor genannten Peptiden abgeleitet werden.

Unter den erfindungsgemäßen Peptiden, die einer spezifischen Bindungsstelle entsprechen, sind auch Peptidderivate zu verstehen, in denen eine oder mehrere Aminosäuren durch eine chemische Reaktion derivatisiert worden sind. Beispiele von erfindungsgemäßen Peptidderivaten sind insbesondere solche Moleküle, in denen das Backbone oder/und reaktive Aminosäureseitengruppen, zum Beispiel freie Aminogruppen, freie Carboxylgruppen oder/und freie Hydroxylgruppen, derivatisiert worden sind. Spezifische Beispiele für Derivate von Aminogruppen sind Sulfonsäure- oder Carbonsäureamide, Thiourethanderivate und' Ammoniumsalze, zum Beispiel Hydrochloride. Carboxylgruppenderivate sind Salze, Ester und Amide. Beispiele für Hydroxylgruppenderivate sind O-Acyl- oder O-Alkylderivate. Die Herstellung der Peptide erfolgt bevorzugt durch chemische Synthese nach dem Fachmann bekannten Methoden und bedürfen hier keiner besonderen Erläuterung. Prinzipiell können die Peptide auch mittels rekombinanter Methoden hergestellt werden. Jedoch tendieren längere Polypeptide oft zur Dimerisierung bzw. Polymerisierung, so daß die Peptide bevorzugt durch chemische Synthese erzeugt werden, um die monomeren Eigenschaften sicher zu stellen.

Weiterhin umfaßt der Begriff Peptidderivat auch solche Peptide, in denen eine oder mehrere Aminosäuren durch natürlich vorkommende oder nicht-natürlich vorkommende Aminosäurehomologe der 20 "Standard"-Arninosäuren ersetzt werden. Beispiele für solche Homologe sind 4-Hydroxyprolin, 5-Hydroxylysin, 3-Methylhistidin, Homoserin, Omithin, β-Alanin und 4-Aminobuttersäure. Die Peptidderivate müssen eine im wesentlichen äquivalente Spezifität oder/und Affinität der Bindung an die zu bestimmenden IgG-Antikörper aufweisen, wie die Peptide, aus denen sie abgeleitet sind.

Als erfindungsgemäße Peptide, die einer spezifischen Bindungsstelle entsprechen, werden auch peptidmimetische Substanzen, im folgenden Peptidmimetika genannt, zu verstehen, die eine im wesentlichen äquivalente Spezifität oder/und Affinität der Bindung an die zu bestimmenden IgG-Antikörper aufweisen, wie die zuvor genannten Peptide oder Peptidderivate. Peptidmimetika sind Verbindungen, die Peptide in ihrer Wechselwirkung mit dem zu bestimmenden Antikörper ersetzen können und gegenüber den nativen Peptiden eine erhöhte Stabilität, insbesondere gegenüber Proteinasen oder Peptidasen, aufweisen können. Methoden zur Herstellung von Peptidmimetika sind beschrieben bei Giannis und Kolter, Angew. Chem. 105 (1993), 1303-1326 und Lee et al., Bull. Chem. Soc. Jpn. 66 (1993), 2006-2010.

Die Länge einer Bindungsstelle, d.h. die Länge des erfindungsgemäßen monomeren Peptids beträgt üblicherweise mindestens 4 Aminosäuren. Bevorzugt liegt die Länge zwischen 4 bis 20,6 bis 15 oder besonders bevorzugt bei 9 bis 12 Aminosäuren. Im Falle der Peptidmimetika oder Peptidderivate ist eine analoge Länge beziehungsweise Größe des Moleküls notwendig.

Die erfindungsgemäßen monomeren Peptide als Bindepartner in monomerer Form enthalten das Epitop, an das der zu bestimmenden IgG-Antikörper spezifisch bindet Am N-terminalen und/oder am C-terminalen Ende des Peptids können jedoch noch weitere flankierende Peptidsequenzen vorhanden sein, die nicht mehr dem spezifischen Epitop entsprechen. Des weiteren ist es möglich, das Peptid mit dem Fachmann geläufigen Spacer-Gruppen zu versehen. Die einzigen Bedingungen sind, daß das Peptid als Bindepartner in monomerer Form tatsächlich monomer vorliegt und die Bindefähigkeit an die zu bestimmenden IgG-Antikörper erhalten bleibt.

Ein weiterer Bestandteil des Rezeptors R₂ ist die Markierung. Bevorzugt wird als Markierung eine direkt nachweisbare Substanz, beispielsweise eine chemilumineszierende, fluoreszierende oder radioaktive Substanz oder ein Metallsol-, Latex- oder Goldpartikel eingesetzt. Weiterhin bevorzugt als Markierung sind Enzyme oder andere biologische Moleküle wie beispielsweise Haptene. Unter den Haptenen ist Digoxigenin eine besonders bevorzugte Markierung. Die Verfahren zur Markierung sind dem Fachmann geläufig und bedürfen hier keiner weiteren Erklärung. Der Nachweis der Markierung erfolgt in an sich bekannter Weise direkt durch Messung der chemilumineszeirenden, fluoreszierenden oder radioaktiven Substanz oder des Metallsol-, Latex- oder Goldpartikels oder durch Messung des durch das Enzym umgesetzten Substrats.

Der Nachweis der Markierung kann auch auf indirekte Weise erfolgen. Dabei bindet ein weiterer Rezeptor, der selbst wiederum mit einer signalerzeugenden Gruppe gekoppelt ist, spezifisch an die Markierung von R₂, bespielsweise ein Hapten wie Digoxigenin. Der Nachweis der signalerzeugenden Gruppe, beispielsweise eine chemilumineszierende, fluoreszierende oder radioaktive Substanz oder ein Enzym oder Goldpartikel, erfolgt nach dem Fachmann geläufigen Methoden. Als weiterer Rezeptor kann beispielsweise ein Antikörper oder ein Antikörperfragment eingesetzt werden, der spezifisch an die Markierung von R₂ bindet. Wird dieser indirekte Nachweis der Markierung verwendet, so ist die Markierung von R₂ bevorzugt Digoxigenin oder ein anderes Hapten, und der Nachweis erfolgt über einen mit Peroxidase gekoppelten Antikörper, der gegen Digoxigenin oder gegen das Hapten gerichtet ist.

Wesentlicher Bestandteil von Rezeptor R₁ ist ein Bindepartner, der mit dem zu bestimmenden IgG-Antikörper spezifisch bindefähig ist. Der Rezeptor R₁ kann direkt an die Festphase gebunden oder mit der Festphase bindefähig sein. Als mit dem zu bestimmenden IgG-Antikörper spezifisch bindefähiger Bindepartner wird ein Bindepartner in monomerer Form wie in Rezeptor R₂ eingesetzt. Entscheidend ist, dass die spezifische Bindefähigkeit des Bindepartners mit den zu bestimmenden IgG-Antikörper erhalten bleibt. Bevorzugt werden jedoch auch für den Rezeptor R₁ Peptide eingesetzt. Die in R₁ enthaltenen Peptide werden nach den gleichen Methoden hergestellt wie die Peptide für R₂.

Die in den Rezeptor R₁ und R₂ enthaltenen antikörperspezifischen Bindepartner bzw. Peptide können identisch oder verschieden sein, müssen aber beide gleichzeitig mit dem zu bestimmenden IgG-Antikörper bindefähig sein.

R₁ kann entweder direkt an die Festphase gebunden sein. Die direkte Bindung von R₁ an die Festphase erfolgt nach dem Fachmann bekannten Methoden. Die Bindung von R₁ an die Festphase kann auch indirekt über ein spezifisches Bindungssystem erfolgen. In diesem Fall ist R₁ ein Konjugat, das aus einem oben erläuterten Peptid und einem Reaktionspartner eines spezifischen Bindungssystems besteht. Unter einem spezifischen Bindungssystem werden hier zwei Partner verstanden, die spezifisch miteinander reagieren können. Das Bindungsvermögen kann dabei auf einer immunologischen Reaktion oder auf einer anderen spezifischen Reaktion beruhen. Bevorzugt wird als spezifisches Bindungssystem eine Kombination von Biotin und Avidin oder Biotin und Streptavidin verwendet. Weitere bevorzugte Kombinationen sind Biotin und Antibiotin, Hapten und Anti-Hapten, Fc-Fragment eines Antikörpers und Antikörper gegen dieses Fc-Fragment oder Kohlenhydrat und Lectin. Einer der Reaktionspartner dieses spezifisch bindefähigen Paares ist dann Teil des Konjugates, das den Rezeptor R₁ bildet.

Der andere Reaktionspartner des spezifischen Bindungssystems liegt in fester Phase vor. Die Bindung des anderen Reaktionspartners des spezifischen Bindungssystems an ein unlösliches Trägermaterial kann nach den üblichen, dem Fachmann bekannten Methoden vorgenommen werden. Hierbei ist sowohl eine kovalente als auch eine adsorptive Bindung geeignet. Als feste Phase besonders geeignet sind Reagenzgläschen oder Mikrotiterplatten aus Polystyrol oder ähnlichen Kunststoffen, die an der Innenoberfläche mit Reaktionspartner des spezifischen Bindungssystems beschichtet sind. Weiterhin geeignet und besonders bevorzugt sind teilchenförmige Substanzen, wie beispielsweise Latexpartikel, Molekularsiebmaterialien, Glaskörperchen, Kunststoffschläuche und dergleichen. Auch poröse, schichtförmige Träger wie Papier können als Träger verwendet werden.

In einer bevorzugten Ausführungsform des erfindungsgemäßen Verfahrens wird als R₁ ein Konjugat aus einem Bindepartner in monomerer Form und einem Reaktionspartner des spezifischen Bindungssystems eingesetzt. In dieser bevorzugten Ausführungsform werden die Rezeptoren R₁ und R₂, sowie die Probe, die den zu bestimmenden IgG-Antikörper enthält, gemeinsam inkubiert. Dabei reagieren die Peptidteile der Rezeptoren R₁ und R₂ spezifisch mit den zu bestimmenden IgG-Antikörpern. Die Bindung dieses Komplexes aus R₁, Probenantikörper und R₂ an die Festphase, die mit dem anderen Reaktionspartner des spezifischen Bindungssystems beschichtet ist, erfolgt über den Reaktionspartner des spezifischen Bindungssystems, der Bestandteil von R₁ ist. Somit ist der gesamte Komplex aus R₁, Probenantikörper und R₂ an die Festphase gebunden. Nach der Trennung der Festphase von der flüssigen Phase und gegebenenfalls Waschung der festen Phase wird die Markierung von R₂ nach dem Fachmann bekannten Methoden nachgewiesen. Durch diese Testfühnmg ist es möglich, einen spezifischen IgG-Antikörper in Anwesenheit von IgM-Antikörpem gleicher Spezifität nachzuweisen.

In einer weiteren bevorzugten Ausführungsform des erfindungsgemäßen Verfahrens wird neben den Rezeptoren R₁ und R₂ noch ein weiterer Rezeptor eingesetzt. In dieser Testführung wird als R₁ ein Konjugat aus einem Bindepartner in monomerer Form und einem Reaktionspartner eines spezifischen Bindungssystems wie beispielsweise Biotin eingesetzt. Hierzu werden die Rezeptoren R₁ und R₂ sowie die Probe, die den zu bestimmenden IgG-Antikörper enthält, gemeinsam inkubiert. Dabei reagieren die Peptidteile der Rezeptoren R₁ und R₂ spezifisch mit den zu bestimmenden IgG-Antikörpern. Über den einen Reaktionspartner eines spezifischen Bindungssystems, der Bestandteil von R₁ ist, erfolgt die Bindung an die Festphase, die mit dem anderen Reaktionspartner des spezifischen Bindungssystems (beispielsweise mit Streptavidin) beschichtet ist. Somit ist der gesamte Komplex aus R₁, R₂, und Probenantikörper an die Festphase gebunden. Nach der Trennung der Festphase von der flüssigen Phase und gegebenenfalls Waschung der festen Phase wird der festphasengebundene Komplex mit einem weiteren Rezeptor inkubiert, der spezifisch die Markierung von R₂ erkennt. Der weitere Rezeptor ist mit einer signalerzeugenden Gruppe, beispielsweise einem Enzym gekoppelt. Nach gegebenenfalls einem weiteren Wasch-Schritt erfolgt der Nachweis des Probenantikörpers über die signalerzeugende Gruppe, in diesem Fall durch das von Enzym umgesetzte Substrat. Wird diese Testführung eingesetzt, so wird als Markierung von R₂ bevorzugt Digoxigenin verwendet. Der weitere Rezeptor besteht in diesem Fall aus einem gegen Digoxigenin gerichteten Antikörper bzw. Antikörperfragment und dem Enzym Peroxidase. Bei dieser Testführung kann die Inkubation der Probe mit R₁, R₂ und dem weiteren Rezeptor auch gleichzeitig erfolgen.

Diese Testführung ist ebenfalls sehr gut für den Einsatz in automatisierten Systemen geeignet, erfordert jedoch zwei oder mehrere Wasch-Schritte. Der große Vorteil dieser Testführung zeigt sich, wenn mehrere antigenspezifische IgG-Antikörper nachgewiesen werden sollen wie z.B. HIV-Antikörper gegen gp41, p17 etc. In einem solchen Fall kann der weitere Rezeptor als Universalmarkierung eingesetzt werden, da dieser weitere Rezeptor spezifisch die Markierung von R₂ erkennt.

Als Proben können alle dem Fachmann bekannten biologischen Flüssigkeiten verwendet werden. Bevorzugt werden als Probe Körperflüssigkeiten wie Vollblut, Blutserum, Blutplasma, Urin, Speichel etc. eingesetzt.

In den Testansätzen können neben der Probe, der Festphase und den aufgeführten Rezeptoren weitere, je nach Anwendung benötigte Zusätze wie Puffer, Salze, Detergenzien, Proteinzusätze wie beispielsweise RSA vorhanden sein. Die notwendigen Zusätze sind dem Fachmann bekannt oder können von ihm auf einfache Weise herausgefunden werden.

Um zu gewährleisten, daß IgM-Antikörper oder Rheumafaktoren den antigenspezifischen IgG-Nachweis nicht stören, können gegebenenfalls zusätzliche Entstörmaßnahmen angewandt werden. Dazu zählen beispielsweise der in der EP-B-0 341 439 offenbarte Einsatz von reduzierenden Substanzen wie Dithiothreitol (DTT), Dithioerythritol (DTE) oder β-Mercaptoethanol. Außerdem können gegebenenfalls Anti-Fd-Antikörper zur Entstörung von Rheumafaktoren eingesetzt werden. Ein solches Konzept ist in der WO 96/14338 offenbart. Die verschiedenen Entstörmaßnahmen können einzeln oder in beliebiger Kombination eingesetzt werden.

Ein weiterer Gegenstand der Erfindung ist ein Reagenz zur Bestimmung eines antigenspezifischen Antikörpers der Immunglobulinklasse G, das neben den üblichen Testzusätzen für Immunoassays wie Puffer, Salze, Detergenzien etc., einen mit dem zu bestimmenden Antikörper bindefähigen Rezeptor R₂ enthält, der aus einem Bindepartner in monomerer Form und einer Markierung besteht.

Ebenfalls ein Gegenstand der Erfindung ist ein Reagenz zur Bestimmung eines antigenspezifischen Antikörpers der Immunglobulinklasse G, das neben den üblichen Testzusätzen für Immunoassays zwei mit dem zu bestimmenden Antikörper bindefähige Rezeptoren R₁ und R₂ enthält, von denen R₁ mit einer Festphase bindefähig ist und R₂ eine Markierung trägt, wobei ein wesentlicher Bestandteil von Rezeptor R₂ ein Bindepartner in monomerer Form ist.

Weiterhin ist ein Gegenstand der Erfindung ein Reagenz zur Bestimmung eines antigenspezifischen Antikörpers der Immunglobulinklasse G, das neben den üblichen Testzusätzen für Immunoassays zwei mit dem zu bestimmenden Antikörper bindefähige Rezeptoren R₁ und R₂ enthält, von denen R₁ mit einer Festphase bindefähig ist und R₂ eine Markierung trägt, wobei ein wesentlicher Bestandteil beider Rezeptoren jeweils Bindepartner in monomerer Form sind.

Des weiteren ist ein Gegenstand der vorliegenden Erfindung die Verwendung von Bindepartnern in monomerer Form zur Bestimmung eines antigenspezifischen Antikörpers der Immunglobulinklasse G.

Die folgenden Beispiele erläutern die Erfindung weiter.

### Beispiele

### 1. Reaktivität mit <HIV 2>MAKs (IgG und IgM) bei Verwendung von monomeren und multimeren Epitopen.

### Beschreibung der Testführung:

Biotin-markierte und Digoxigenin-markierte monomere (Test A) bzw. multimere (Test B) Antigene (HIV 2) reagieren mit Probenantikörpem und Streptavidin-beschichteter Festphase (Inkubation bei 25°C oder 37°C, ca. 60 bis 180 min, in diesem Beispiel: 120 min, 25°C). Nach einem Waschschritt reagiert der wandgebundene Immunkomplex mit anti-Digoxigenin-Peroxidase-Konjugat (Inkubation bei 25°C oder 37°C, ca. 30 bis 120 min, in diesem Beispiel: 60 min 25°C). Nach einem weiteren Waschschritt wird der Peroxidase-Konjugat-markierte Immunkomplex durch eine Substratreaktion nachgewiesen (Konjugat-Inkubation 60 min bei 25°C). Generell kann die Konjugat-Inkubation bei 25°C oder 37°C, für ca. 30 bis 120 min durchgeführt werden.

Die Reaktionsschritte (außer der Substrat-Reaktion) finden statt in einem Tris/HCl Puffer (pH 7.5, 50 bis 150 mM, in diesem Beispiel 100 mM) mit ca. 0.05 bis 0.4 % Detergenz (hier 0.2 % Polidocanol), und ca. 0.5 % Protein/Proteinderivat-Zusätze (hier u.a. Pepton aus Lactalbumin und RSA).

Die Probenantiköper sind in diesem Fall monoklonale Maus-Antiköper (IgM und IgG) gegen ein HIV 2 Epitop verdünnt auf ca. 2 - 20 µg/ml in anti-HIV negativem Humanserum.

**Tabelle 1:**

| Testergebnisse Vergleich Test A und B: | | | | |
|---|---|---|---|---|
| Extinktionen in mE | | | | |
| **Proben** | **Test A (monomere Epitope)** | **Bewertung** | **Test B (multimere Epitope)** | **Bewertung** |
| MAK <HIV 2> **IgG** 20.3.1. | 2764 | positiv | 2800 | positiv |
| MAK <HIV 2> **IgG** 23.5.3. | 9999 | positiv | 3974 | positiv |
| MAK <HIV 2> **IgM** 2.6.6. | 6 | negativ | 3271 | positiv |
| MAK <HIV 2> **IgM** 2.11.7. | 6 | negativ | 4250 | positiv |
| MAK <HIV 2> **IgM** 2.22.8. | 6 | negativ | 6442 | positiv |

Die Verwendung von HIV2-spezifischen Bindepartnern in monomerer Form ermöglicht es, spezifisch IgG-Antikörper gegen HIV2 nachzuweisen. Antikörper der Klasse IgM mit gleicher Antigenspezifität werden nicht erkannt (Test A).

Bei Verwendung von HIV2-spezifischen Bindepartnem in multimerer Form ist die Diskriminierung zwischen IgG und IgM nicht möglich. (Test B).

### 2. Reaktivität mit Serum-Antiköpern einer HIV 2-Serokonversion eines Schimpansen

Experimentelle Durchführung wie Beispiel 1.

**Tabelle 2:**

| Testergebnisse Schimpansen-Serokonversion mit Test A und B: Extinktionen in mE | | | | |
|---|---|---|---|---|
| **Sequentielle Serum- Proben nach Infektion** | **Test A (monomere Epitope)** | **Bewertung** | **Test B (multimere Epitope)** | **Bewertung** |
| Woche 0 (bei Infektion) Status: keine Antiköper | 6 | negativ | 98 | negativ |
| Woche 1 nach Infektion Status: keine Antikörper | 7 | negativ | 89 | negativ |
| Woche 3 nach Infektion Status: IgM-Antikörper | 6 | negativ | 673 | positiv |
| Woche 7 nach Infektion Status: IgM und IgG Antikörper | 250 | grenzwertig | 589 | positiv |
| Woche 10 nach Infektion Status: IgG Antikörper | 8767 | positiv | 6845 | positiv |

Die Verwendung von Bindepartnem in monomerer Form ermöglicht den Nachweis der Serokonversion nach einer HIV2-Infektion. Der Vorteil von Test A zeigt sich besonders deutlich in Woche 3 nach der Infektion: Es existieren nur IgM-Antikörper, die mit den Bindepartnern in monomerer Form nicht detektiert werden. Erst nach dem Auftreten von IgG-Antikörpern zeigt TestA ein positives Signal. Test B, der multimere Epitope verwendet, ist nicht in der Lage, zwischen IgG und IgM gleicher Spezifität zu unterscheiden.

### 3. Reaktivität mit Serum-Antikörpern einer HIV 1-Serokonversion eines Patienten

Experimentelle Durchführung wie Beispiel 1, aber Antigene von HTV 1.

**Tabelle 3:**

| Testergebnisse HIV 1-Serokonversion mit Test A und B Extinktionen in mE | | | | |
|---|---|---|---|---|
| **Sequentielle Serum- Proben nach erster Untersuchung** | **Test A (monomere Epitope)** | **Bewertung** | **Test B (multimere Epitope)** | **Bewertung** |
| Tag 0 Status: keine Antiköper | 30 | negativ | 89 | negativ |
| Tag 14 Status: keine Antikörper | 28 | negativ | 78 | negativ |
| Tag 26 Status: IgM-Antikörper | 35 | negativ | 9999 | positiv |
| Tag 35 Status: IgM und IgG Antikörper | 250 | grenzwertig | 9999 | positiv |
| Tag 40 Status: IgM und IgG Antikörper | 589 | positiv | 9151 | positiv |

Auch bei einer Infektion mit HIVI ist (analog zu HIV2 gemäß Beispiel 2) mit Test A ein zuverlässiger Nachweis von IgG-Antikörpern möglich.

### 4. Nachweis von IgG gegen Rubella mit Peptiden, die monomere Epitope enthalten

### Beschreibung der Testführung:

Der Nachweis von Rubella-spezifischem IgG mittels der erfindungsgemäßen Bindepartner in monomerer Form wird an einem Elecsys® 2010-Gerät der Boehringer Mannheim GmbH, Deutschland, gemäß Herstellerangaben durchgeführt. Folgende Reagenzien werden eingesetzt:
- Reagenz R1:: cyclisches Peptid von Rubella E1-Antigen, biotinyliert. Tris Puffer, pH 7.5, 0,2 % Myrij, 0.2 % RSA, 0,1 % R-IgG.
- Reagenz R2:: cyclisches Peptid von Rubella E1-Antigen, ruthenyliert. Tris Puffer, pH 7.5, 0,2 % Myrij, 0.2 % RSA, 0,1 % R-IgG.

Als Proben werden humane Serumproben eingesetzt.

Die Durchführung des Tests erfolgt in den Schritten:
1. 30 µl Probe + 65 µl Rl + 65 µl R2
2. Inkubation bei 37°C, 9 min
3. Zugabe von 40 µl SA-beschichteten magnetischen Beads
4. Inkubation bei 37°C, 9 min
5. Detektionsreaktion: Messen des Elektrochemilumineszenz-Signals

**Tabelle 4:**

| Ergebnisse | | | |
|---|---|---|---|
| **Proben** | **Charakterisierung** | **counts** | **IU/ml** |
| Nr. 1 | IgM neg., IgG neg. | 1359 | 0,5 |
| Nr. 3 | IgM neg., IgG neg. | 1370 | 0,5 |
| Nr. 8 | IgM neg., IgG neg. | 1338 | 0,5 |
| Nr. 1.3 | IgM pos., IgG neg. | 929 | 0,1 |
| Nr. 1.4 | IgM pos., IgG neg. | 981 | 0,2 |
| Nr. 255 | IgM neg., IgG pos. | 11256 | 83 |
| Nr. 272 | IgM neg., IgG pos. | 26254 | 192 |
| Nr. 278 | IgM neg., IgG pos. | 4453 | 32 |
| Nr. 283 | IgM neg., IgG pos. | 49328 | 363 |

Es werden nur Proben, die antigenspezifisches IgG enthalten, als positiv erkannt Proben, die nur antigenspezifisches IgM enthalten (Nr. 1.3 und 1.4) werden erfindungsgemäß nicht als positiv erkannt.

## Patentansprüche

1. Verfahren zur Bestimmung eines antigenspezifischen Antikörpers der Immunglobulinklasse G in einem Brückentest nach dem Doppelantigen-Testprinzip durch Inkubation der Probe mit mindestens zwei verschiedenen Rezeptoren R₁ und R₂, wobei beide Rezeptoren mit den Antigenbindestellen des Antikörpers spezifisch bindefähig sind, R₁ an eine feste Phase gebunden ist oder gebunden werden kann und R2 eine Markierung trägt, **dadurch gekennzeichnet, dass** man als R₂ ein Konjugat aus einem vom zu bestimmenden Antikörper spezifisch erkannten Bindepartner in monomerer Form und einer Markierung verwendet und dass ein wesentlicher Bestandteil von R₁ ein Bindepartner in monomerer Form ist.

2. Verfahren nach Anspruch 1, **dadurch gekennzeichnet, dass** als spezifisches Bindungssystem für die Bindung von R₁ an die Festphase Biotin/Avidin, Biotin/Streptavidin, Biotin/Antibiotin, Hapten/Antihapten, Fc-Fragment eines Antikörpers/Antikörper gegen dieses Fc-Fragment, Kohlenhydrat/Lectin verwendet wird.

3. Verfahren nach einem der Ansprüche 1 oder 2, **dadurch gekennzeichnet, dass** der Rezeptor R₂ mit einer chemilumineszierenden, fluoreszierenden oder radioaktiven Substanz, einem Enzym oder einem anderen biologischen Molekül markiert ist.

4. Verfahren nach einem der Ansprüche 1 bis 3, **dadurch gekennzeichnet, dass** man die Probe gleichzeitig mit R₁ und R₂ inkubiert.

5. Verfahren nach einem der Ansprüche 1 bis 4, **dadurch gekennzeichnet, dass** der Testansatz mit einem weiteren Rezeptor, der spezifisch an die Markierung des Rezeptors R₂ bindet, inkubiert wird, wobei der weitere Rezeptor ein Konjugat aus einem für die Markierung von R₂ spezifischen Rezeptor und einer Markierung ist, und anschließend die Markierung bestimmt wird.

6. Verfahren nach einem der Ansprüche 1 bis 5, **dadurch gekennzeichnet, dass** die Konzentrationsverhältnisse von R₁:R₂ von 0,5:1,0 bis 1,0:5,0 betragen.

7. Reagenz zur Bestimmung eines antigenspezifischen Antikörpers der Immunglobulinklasse G nach einem der vorhergehenden Ansprüche, **dadurch gekennzeichnet, dass** es zwei mit dem zu bestimmenden Antikörper bindefähigen Rezeptoren R₁ und R₂ enthält, von denen R₁ mit einer Festphase bindefähig ist und R₂ eine Markierung trägt, wobei ein wesentlicher Bestandteil beider Rezeptoren jeweils Bindepartner in monomerer Form sind.

## Claims

1. Method for the determination of an antigen-specific antibody of the immunoglobulin G class in a bridge test using a double antigen test principle by incubating the sample with at least two different receptors R₁ and R₂ in which both receptors are capable of binding specifically to the antigen binding sites of the antibody, R₁ is bound to a solid phase or can be bound to a solid phase and R₂ carries a label, wherein a conjugate of a binding partner in monomeric form which is specifically recognized by the antibody to be determined and a label is used as R₂ and an essential component of R₁ is a binding partner in monomeric form.

2. Method as claimed in claim 1, wherein biotin/avidin, biotin/streptavidin, biotin/antibiotin, hapten/antihapten, Fc fragment of an antibody/antibody against this Fc fragment, carbohydrate/lectin is used as the specific binding system for binding R₁ to the solid phase.

3. Method as claimed in one of the claims 1 to 2, wherein the receptor R₂ is labelled with a chemiluminescent, fluorescent or radioactive substance, an enzyme or another biological molecule.

4. Method as claimed in one of the claims 1 to 3, wherein the sample is simultaneously incubated with R₁ and R₂.

5. Method as claimed in one of the claims 1 to 4, wherein the test mixture is incubated with an additional receptor which specifically binds to the label of the receptor R₂, wherein the additional receptor is a conjugate of a receptor that is specific for the label of R₂ and a label, and the label is subsequently determined.

6. Method as claimed in one of the claims 1 to 5, wherein the concentration ratios of R₁ : R₂ are 0.5 : 1.0 to 1.0 : 5.0.

7. Reagent for determining an antigen-specific antibody of the immunoglobulin G class as claimed in one of the previous claims, wherein it contains two receptors R₁ and R₂ capable of binding to the antibody to be determined of which R₁ can bind to a solid phase and R₂ carries a label and an essential component of both receptors is in each case a binding partner in monomeric form.

## Revendications

1. Procédé de détermination d'une anticorps spécifique à un antigène de la classe des immunoglobulines G dans un test de pontage selon le principe de test à antigène double par incubation de l'échantillon avec au moins deux récepteurs différents R₁ et R₂, les deux récepteurs étant capables de se lier spécifiquement au site de fixation d'antigène de l'anticorps, R₁ étant lié ou pouvant être lié à une phase solide, et R₂ portant un marqueur, **caractérisé en ce que** l'on emploie comme R₂ un conjugué d'un partenaire de liaison reconnu spécifiquement par l'anticorps à déterminer, sous forme monomère et d'un marqueur, et **en ce que** un composant essentiel de R₁ est un partenaire de liaison sous forme monomère.

2. Procédé selon la revendication 1, **caractérisé en ce que** l'on emploie en tant que système de liaison spécifique pour fixation de R₁ à la phase solide, le système biotinelavidine, biotine/streptavidine, biotine/antibiotine, haptène/antihaptène, fragment Fc d'un anticorps/anticorps contre ce fragment Fc, glucide/lectine.

3. Procédé selon l'une quelconque des revendications 1 ou 2, **caractérisé en ce que** le récepteur R₂ est marqué par une substance chimiluminescente, fluorescente ou radioactive, une enzyme ou une autre molécule biologique.

4. Procédé selon l'une quelconque des revendications 1 à 3, **caractérisé en ce que** l'on incube simultanément l'échantillon avec R₁ et R₂.

5. Procédé selon l'une quelconque des revendications 1 à 4, **caractérisé en ce que** le mélange de test est incubé avec un récepteur supplémentaire qui se fixe spécifiquement au marqueur du récepteur R₂, le récepteur supplémentaire étant un conjugué d'un récepteur spécifique au marqueur de R₂ et d'un marqueur, puis le marqueur est déterminé.

6. Procédé selon l'une quelconque des revendications 1 à 5, **caractérisé en ce que** les rapports de concentration R₁:R₂ vont de 0,5:1,0 à 1,0:5,0.

7. Réactif pour déterminer un anticorps spécifique à un antigène de la classe des immunoglobulines G selon l'une quelconque des revendications précédentes, **caractérisé en ce qu'**il contient deux récepteurs R₁ et R₂, capables de se fixer sur l'anticorps à déterminer, parmi lesquels R₁ est capable de se lier à une phase solide et R₂ porte un marqueur, des partenaires de liaison sous forme monomère constituent respectivement un composant essentiel des deux récepteurs.
